Europäisches Patentamt

European Patent Office

Office européen des brevets

Veröffentlichungsnummer: **0 302 990**

**A1**

## EUROPÄISCHE PATENTANMELDUNG

(21) Anmeldenummer: 88103377.3

(22) Anmeldetag: 04.03.88

(51) Int. Cl.⁴ **A61M 5/31** , **A61M 5/14**

| | |
|---|---|
| Die Bezeichnung der Erfindung wurde geändert (Richtlinien für die Prüfung im EPA, A-III, 7.3). | (71) Anmelder: **B. Braun Melsungen AG** **Carl-Braun Strasse** **D-3508 Melsungen(DE)** |
| (30) Priorität: **23.04.87 DE 3713551** | (72) Erfinder: **Scherer, Jürgen** **Birnheck 12** **D-6233 Kelkheim 3-Ruppertshain(DE)** |
| (43) Veröffentlichungstag der Anmeldung: **15.02.89 Patentblatt 89/07** | (74) Vertreter: **Selting, Günther, Dipl.-Ing. et al** **Patentanwälte von Kreisler, Selting, Werner** |
| (84) Benannte Vertragsstaaten: **DE ES FR GB IT NL SE** | **Deichmannhaus am Hauptbahnhof** **D-5000 Köln 1(DE)** |

(54) **Injektionsstück für eine medizinische Flüssigkeitsübertragungsleitung.**

(57) Die Erfindung bezieht sich auf ein Injektionsstück für eine Leitung (11a, 11b) eines medizinischen Flüssigkeitsübertragungssystems mit einem Hohlkörper, der einen Wandteil (15) aus durchstechbarem, gummielastischem Material aufweist und mit einem Anschlußkonus (12) mit Durchlaßkanal (26) zur Verbindung mit einem Gegenelement (19) der Leitung (11a, 11b) versehen ist. Dabei ist der Anschlußkonus (12) mit einem Käfig (13) fest verbunden, der einen gegen den Durchlaßkanal (26) des Anschlußkonus (12) offenen, gummielastischen, perforierbaren Schlauchkörper (15) abstützt.

Ein solches Injektionsstück kann mit Hilfe des untrennbar vorgesehenen Anschlußkonus (12) druckdicht mit der Leitung (11a, 11b) verbunden werden und es ermöglicht eine allseitige Zuspritzung durch den den durchstechbaren Wandteil bildenden Schlauchkörper (15). Beim Einführen einer Stahlkanüle in den Schlauchkörper (15) muß die Leitung (11a, 11b) des Flüssigkeitsübertragungssystems nicht verdreht werden, weil das Injektionsstück eine Zuspritzung von allen Seiten ermöglicht.

FIG.1

## Injektionsstück

Die Erfindung bezieht sich auf ein Injektionsstück für eine Leitung eines medizinischen Flüssigkeitsübertragungssystems mit einem Hohlkörper, der einen Wandteil aus durchstechbarem, gummielastischem Material aufweist und mit einem Anschlußkonus mit Durchlaßkanal zur Verbindung mit einem Gegenelement der Leitung versehen ist.

Bei medizinischen Flüssigkeitsübertragungssystemen zur Überleitung von Infusions- oder Transfusionslösungen und bei Venenpunktionssystemen werden Injektionsstücke verwendet, um der Lösung oder der punktierten Vene flüssige Medikamente o. dgl. zuspritzen zu können. Hierzu wird der durchstechbare Wandteil mit der Stahlkanüle einer Spritze durchstochen und aus der Spritze wird das Injektat in die zum Patienten führende Leitung injiziert. Anschließend wird die Stahlkanüle wieder aus dem Wandteil herausgezogen, der infolge seiner Elastizität die Perforationsstelle selbsttätig schließt. Mit Hilfe des Anschlußkonus ist der Hohlkörper des Injektionsstückes an ein Gegenelement der Leitung angekoppelt, wobei der Außenkonus in üblicher Weise mit Abstand von einer Gewindehülse umgeben sein kann, die mit radialen Vorsprüngen an dem Gegenelement der Leitung eine Verriegelung der Verbindung bewirkt (Luer-Lock-Verbindung).

Es ist bekannt, den Hohlkörper aus einem an beiden Enden offenen, flexiblen Latex-Schlauchstück oder -Formstück herzustellen, so daß er insgesamt gummielastisch und durchstechbar ist. Der Anschlußkonus zur Verbindung mit einem Gegenelement der zum Patienten führenden Leitung ist in diesem Fall ein separater Teil, der mit Hilfe eines in das eine aufweitbare Ende des Schlauchstückes eingesteckten Konnektorstutzens mit dem Schlauchstück verbunden ist. In das andere aufweitbare Ende des Schlauchstückes wird das Ende einer z.B. von einer Tropfkammer eines Infusionsgerätes kommenden Schlauchleitung dicht eingesteckt. Zwar ist ein solches Injektionsstück insbesondere zum endständigen Zuspritzen von flüssigen Präparaten in eine Flüssigkeitsübertragungsleitung günstig, da die Einspritzung in das Schlauchstück von allen Seiten erfolgen kann und die Leitung nicht verdreht wird. Es haben sich jedoch Dichtigkeitsprobleme ergeben, weil die nur von der elastischen Klemmkraft des Schlauchstückes abhängigen Steckverbindungen an den Enden des Schlauchstückes nicht druckbeständig sind. Ferner besteht die Gefahr, daß beim Einstechen der Spritzenkanüle in die Wand des Schlauchstückes Partikel ausgestanzt werden, die ohne Filterungsmöglichkeit in die zum Patienten führende Leitung gelangen.

Ferner sind Injektionszwischenglieder mit einem starren Gehäuse bekannt, das einen geraden Durchlaßkanal mit zwei entgegengesetzt gerichteten Schlauchansätzen zum Aufstecken von Schlauchleitungsenden aufweist und das einen mit dem Durchlaßkanal in Verbindung stehenden Querstutzen trägt, der von einer durchstechbaren Membran abdichtend verschlossen ist. Infolge des starren Gehäuses werden die Verbindungen zwischen Injektionszwischenglied und Schlauchleitungsenden durch den beim Zuspritzen durch den Querstutzen entstehenden Druck nicht merklich beeinträchtigt. Zur endständigen Zuspritzung, d.h. zur Anbringung des Injektionszwischengliedes am patientenseitigen Ende einer Infusions- oder Transfusionsleitung ist ein starres Gehäuse mit sehr kleiner Zuspritzzone allerdings nicht gut geeignet, weil es zum Zuspritzen jeweils in eine günstige Position gedreht werden muß. Hierdurch kann sowohl die Dichtigkeit der Verbindungen leiden als auch eine Beeinträchtigung des Flüssigkeitsstromes hervorgerufen werden. Im übrigen ist die Kleinheit der durchstechbaren Membran ungünstig, weil bei mehrmaligem Zuspritzen keine neuen Einstechstellen auswählbar sind und die Schließfähigkeit des Materials leidet.

Auch ist ein Injektionsstopfen zur lösbaren Verbindung mit einem Gegenelement an einer Schlauchleitung eines Venenpunktionssystems zur intermittierenden Injektion und wiederholten Blutentnahme bekannt (DE-GM 77 27 563). Dieser Verschlußstopfen besteht aus einem zylindrischen Gehäuse, das an einem Ende einen Anschlußkonus mit Durchlaßkanal aufweist und das am anderen Ende mit einer durchstechbaren Membran versehen ist, die den Durchlaßkanal abdichtend verschließt. Zum Zuspritzen eines Medikamentes durch die Membran in die zum Patienten führende Leitung muß auch in diesem Falle der Injektionsstopfen und mit ihm die Schlauchleitung manipuliert werden, um die Membran in geeignete Position in Bezug auf die Spritze zu bringen. Hierdurch und durch die Kleinheit der Membran ergeben sich die bei dem Injektionszwischenstück mit Zuspritzmembran geschilderten Nachteile.

Gemäß DE-OS 31 36 605 ist ein Zuspritzventil bekannt, bei dem der elastische Wandteil des Hohlkörpers nicht durchstechbar ist, sondern eine Schlitzung enthält, die mit Hilfe des Anschlußkegels einer Spritze öffenbar ist, um Flüssigkeit in den Hohlkörper einzuspritzen. Da die Schlitzung sich im Bereich eines seitlichen Ansatzstutzens befindet, muß auch dieses Zuspritzventil zur Betätigung jeweils in eine günstige Position gebracht werden, was zu einer Verdrehung oder sogar Abknickung der Schlauchleitung führen kann, in die das Zuspritzventil eingesetzt ist. Außerdem besteht bei diesem Zuspritzventil nach mehrmaliger Benutzung die Gefahr, daß die Spannkraft des elastischen Materials nachläßt und unzureichende Zusammenpressung der Dichtlippen Undichtigkeiten an der Schlitzung zur Folge hat. Auch kann der nach einer Zuspritzung zwischen den Anlageflächen der Dichtlippen zurückbleibende Flüssigkeitsfilm eine Kontaminationsgefahr für die nachfolgende Zuspritzung bedeuten.

Der Erfindung liegt die Aufgabe zugrunde, ein Injektionsstück der eingangs genannten Art zu schaffen, das drucksicher mit der Leitung eines medizinischen Flüssigkeitsübertragungssystems verbindbar ist, und das eine allseitige Zuspritzung auf einem ringförmigen Umfangsbereich ermöglicht.

Zur Lösung dieser Aufgabe ist erfindungsgemäß vorgesehen, daß der Anschlußkonus mit einem korbartigen, durchbrochen ausgebildeten Käfig fest verbunden ist, der gegen den Wandteil in Form eines gegen den Durchlaßkanal des Anschlußkonus offenen, gummielastischen, perforierbaren Schlauchkörpers abstützend anliegt.

Ein solches Injektionsstück kann bei Ausbildung als den Durchlaßkanal verschließender Injektionsstopfen oder als in eine Leitung einsetzbares Injektionszwischenglied mit Hilfe des untrennbar vorgesehenen Anschlußkonus druckdicht mit einer Leitung verbunden werden und es ermöglicht eine allseitige Zuspritzung durch den den durchstechbaren Wandteil bildenden Schlauchkörper. Beim Einführen einer Stahlkanüle in den Schlauchkörper wird die Leitung des Flüssigkeitsübertragungssystems nicht verdreht, weil das Injektionsstück eine Zuspritzung von allen Seiten ermöglicht. Dies ist für den Anwender bequem und garantiert einen kontinuierlichen Flüssigkeitsstrom durch die Leitung. Der Anschlußkonus kann mit radialem Abstand von einer Gewindehülse umgeben sein, die mit Verriegelungselementen an dem Gegenelement (Innenkonus mit radialen Ansätzen) der Leitung zusammenwirkt, wodurch sich eine sogenannte LuerLockVerbindung ergibt, die sicher ist und ein einfaches Austauschen des Injektionsstückes mit anderen Gegenelementen ermöglicht.

Anschlußkonus und Käfig sind vorteilhafterweise zwei miteinander verschweißte Teile aus starrem Kunststoff. Der Schlauchkörper ist als Gummiformstück ausgebildet, das der äußeren oder inneren Form des Käfigs angepaßt ist. Ränder des Schlauchstückes können mit Teilen des Käfigs und/oder des Anschlußkonus verklebt sein, so daß der Hohlraum in dem Injektionsstück nach außen dicht ist.

Vorteilhafterweise ist vorgesehen, daß der Käfig becherförmig mit mehreren in gleichem Abstand umfangsmäßig verteilten Axialrippen ausgebildet ist und daß der Schlauchkörper formschlüssig in den Käfig eingespannt ist. Der Schlauchkörper wird auf diese Weise gegen Handberührung von außen geschützt. Außerdem begünstigt die Spannkraft der Andrückung des Schlauchkörpers gegen den Käfig ein gerades, ablenkungsfreies Durchstechen des Schlauchkörpers mit der Stahlkanüle und bewirkt eine verbesserte Selbstschließung der von der Stahlkanüle erzeugten kleinen Perforationsschlitze in dem Schlauchkörper.

Bei Verwendung des Injektionsstückes als Injektionszwischenglied, das in eine Leitung eines Flüssigkeitsübertragungssystems eingesetzt wird, weist der Käfig an seinem dem Anschlußkonus gegenüberliegenden Ende einen Konnektorstutzen auf, und es ist in dem dem Konnektor stutzen zugewandten Ende des Schlauchkörpers eine Öffnung ausgebildet. Der Schlauchkörper ist in diesem Falle an beiden axialen Enden offen, so daß die Flüssigkeit frei durch den Konnektorstutzen, den Hohlraum des Injektionsstückes und den Anschlußkonus hindurchströmen kann. Die Öffnungsränder sind dicht mit Teilen des Käfigs bzw. Anschlußkonus verbunden.

Alternativ kann der Schlauchkörper an seinem dem Anschlußkonus abgewandten Ende geschlossen ausgebildet sein. Durch eine solche Gestaltung ist das Injektionsstück als Infusionsstopfen zum Abschluß einer Leitung mit Zuspritz- oder Entnahmemöglichkeit verwendbar. Der Käfig ist dabei vorteilhafterweise in seinem Scheitelbereich offen, so daß die geschlossene Kuppelfläche des Schlauchkörpers freiliegt. Auf diese Weise kann die Stahlkanüle sowohl umfangsmäßig zwischen den Axialrippen hindurch als auch axial von der Stirnseite des Injektionsstückes her eingeführt werden. Die vielseitige Einspritzmöglichkeit bei einem solchen Injektionsstück erleichtert die Handhabung bei der Zuspritzung bzw. Blutentnahme und verringert die Gefahr der Lochbildung durch Mehrfach-Perforation an der gleichen Stelle, weil die zum Durchstechen verfügbare Oberfläche des Schlauchkörpers groß ist.

Sowohl bei der Ausbildung des Injektionsstückes als Zwischenstück als auch bei seiner Gestaltung als Verschlußstopfen mit Punktionsmöglichkeit kann zwischen dem Anschlußkonus und dem Schlauchkörper ein Filter angeordnet sein. Der Filter befindet sich jenseits des durchstechbaren Schlauchkörpers, so daß er von der Injektionskanüle nicht versehentlich getroffen werden kann und seine Aufgabe zuverlässig erfüllt. Diese Aufgabe besteht darin, Partikel auszufiltern, die aus der Spritze selbst oder aus der Glasampulle stammen, aus der die Spritze aufgezogen worden ist. Außerdem soll er Partikel aus der zum Patienten führenden Leitung fernhalten, die beim Durchstechen der Spritzenkanüle durch den gummielastischen Schlauchkörper durch Abrieb oder Ausstanzung entstehen. Dies ist eine erhebliche Verbesserung gegenüber dem erwähnten herkömmlichen Latex-Formstück, das am Ende einer Infusionsleitung angebracht ist. Hierbei kann üblicherweise nur ein am Ende der Tropfkammer angeordneter Filter zum Einsatz kommen, der lediglich die Lösung bzw. die in die Infusionsflasche zugespritzten Medikamente filtriert, so daß die bei der endständigen Zuspritzung in die Leitung eingeführten Partikel ungehindert zum Patienten gelangen können. Für eine vor dem Patienten unmittelbar angebrachte Filtrierungsmöglichkeit muß dann wiederum ein separater Filter angeordnet werden. Dies ist wegen der zahlreichen Anschlußstellen in der Leitung bei der Montage unbequem und kann Anlaß für Undichtigkeiten sein.

In vorteilhafter Weise ist erfindungsgemäß der Filter als hohlzylindrischer Flächenfilter ausgebildet und in einem mit dem Anschlußkonus verbundenen Gehäuseteil untergebracht. Der hohlzylindrische Flächenfilter ist günstig, weil durch seine große Filterfläche die Gefahr von Verstopfungen gering ist. Zum Zusammenbau des Injektionsstückes wird der Schlauchkörper in den Käfig eingesetzt und der Filter wird in die Aufnahme des Gehäuses des Anschlußkonus so eingepaßt, daß er axial angeströmt wird, und die Flüssigkeit ihn radial verläßt. Sodann werden Käfig und Anschlußkonus-Gehäuse miteinander verschweißt. Auch können ebene Flächenfilter (Membranfilter, Flachgewebefilter, Tiefenfilter aus non-woven-Materialien) verwendet werden. die z.B. als flache, runde Filter gestaltet sind. Vorteilhaft wären für Injektionslösungen und Infusionslösungen Membranfilter mit 0,2 bis 5μm Porenweite.

In der Zeichnung sind Ausführungsbeispiele der Erfindung schematisch dargestellt. Es zeigen:

Fig. 1 einen Längsschnitt durch ein als Zwischenglied verwendbares Injektionsstück,

Fig. 2 Teilquerschnitt und Teildraufsicht des Injektionsstückes nach Fig. 1,

Fig. 3 einen Längsschnitt durch ein als Zwischenglied verwendbares Injektionsstück mit Filter,

Fig. 4 Teilquerschnitt und Teildraufsicht des Injektionsstückes nach Fig. 3,

Fig. 5 einen Längsschnitt durch ein Injektionsstück ähnlich Fig. 3 mit abgewandelter Filteranordnung,

Fig. 6 einen Längsschnitt durch ein als Verschlußstopfen verwendbares Injektionsstück und

Fig. 7 eine Draufsicht auf das Injektionsstück nach Fig. 5.

Ein Injektionsstück 10, das in eine von einer Tropfkammer zum Patienten führende Schlauchleitung IIa,IIb eingesetzt ist, besteht im wesentlichen aus drei kreiszylindrischen, koaxialen Teilen, und zwar, einem starren Außenkonus 12 mit Durchlaßkanal 26, einem starren Käfig 13 mit Konnektorstutzen 14 und einem in dem Käfig untergebrachten Schlauchkörper 15, der als elastisches Gummiformstück ausgebildet ist. Der Außenkonus 12 ist mit radialem Abstand von einer Hülse 16 umgeben, welche an ihrer Innenseite Gewindeteile 17 aufweist. Die Gewindeteile 17 wirken mit Verriegelungselementen 18 zusammen, die am äußeren Rand eines Gegenelementes 19 an der Leitung IIb radial nach außen vorstehen und an ihrer Unterseite entsprechend der Steigung der Gewindeteile 17 abgeschrägt sind. Die Innenwand 20 des Gegenelementes 19 erweitert sich nach außen konisch und ermöglicht einen passenden abdichtenden Zusammengriff mit dem Außenkonus 12 in zusammengeführtem und verriegeltem Zustand. An das verbreiterte Ende des Außenkonus 12 schließt sich eine radialgerichtete ringförmige Basis 21 an, die den Außenkonus 12 mit der Gewindehülse 16 verbindet. Mit dem äußeren Rand der Basis 21 ist einstückig ein Profilring 22 verbunden, dessen innerer Umfang mit dem Innenumfang der Gewindehülse 16 im wesentlichen fluchtet. Der Profilring 22 bildet einen radialen Flansch mit zwei konzentrischen Stufenabschnitten 23,24, die beide entgegengesetzt zur Gewindehülse 16 gerichtet sind. Der innere Stufenabschnitt 24 kleineren Durchmessers ist in Bezug auf den äußeren Stufenabschnitt 23 angehoben und wird an seinem Innenumfang von einer Ringwulst 25 mit abgerundeter Scheitelzone begrenzt. Der von der Ringwulst 25 umgebene Hohlraum 27 bildet mit dem Durchlaßkanal 26 in dem Außenkonus 12 einen trichterförmigen Flüssigkeits-Führungsteil.

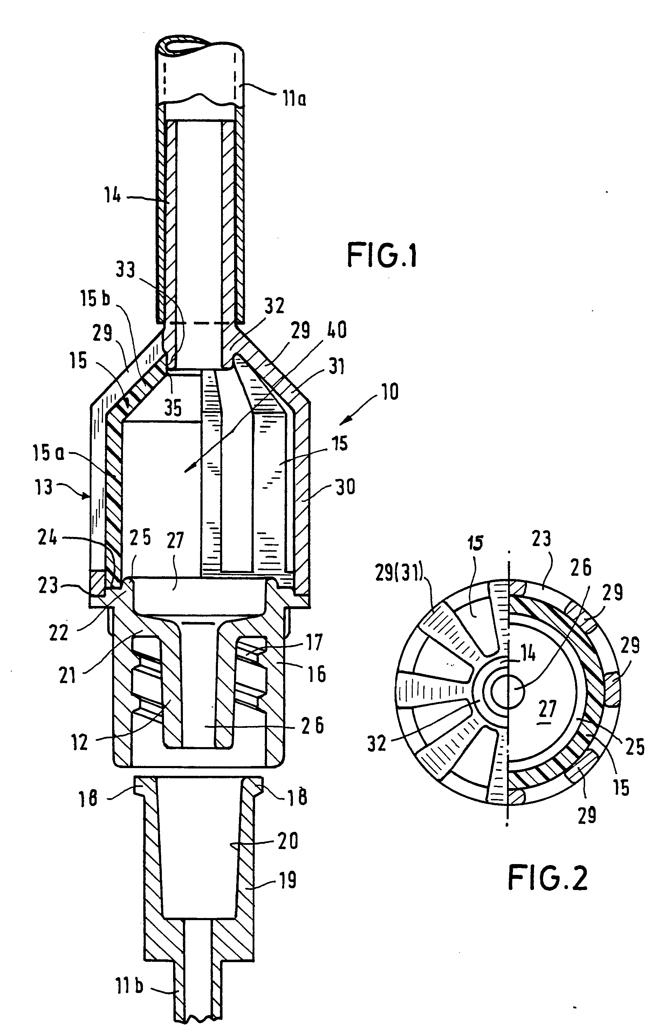

Der aus starrem Kunststoff bestehende Käfig 13 weist umfangsmäßig mit gleichen Abständen angeordnete gerade Axialrippen 29 auf, die sich zu einer Becherform mit einem zylindrischen Abschnitt 30 und einem nach oben verjüngten konischen Abschnitt 31 ergänzen. Die Axialrippen 29 des zylindrischen Abschnittes 30 haben über ihre Länge gleiche Breite. während sie in dem konischen Abschnitt 31 nach oben schmaler werden und mit ihrem schmalsten Ende an einen Ringteil 32 angreifen, von dem der nach außen ragende gerade Konnektorstutzen 14 und ein nach innen gerichteter ringförmiger Stützteil 33 ausgehen. Bei dem dargestellten Beispiel besitzt der Käfig 13 acht Axialrippen 29, die jeweils etwa rechteckigen Querschnitt mit abgerundeten Ecken aufweisen (Fig. 2).

Der Käfig 13 umschließt den einen Innenraum 40 begrenzenden Schlauchkörper 15, dessen Form dem Innenprofil des Käfigs 13 angepaßt ist und dessen Außenfläche gegen die Innenfläche der Axialrippen 29 gleichmäßig anliegt. Infolge der Anpassung erhält der Schlauchkörper 15 einen zylindrischen Teil 15a und einen konischen Teil 15b. Seine gesamte Wand ist geschlossen und ist zwischen den Axialrippen 29 des Käfigs 13 streifenweise von außen frei zugänglich. Der untere kreisförmige Rand des Schlauchkörpers 15 ist in den inneren Stufenabschnitt 24 des Profilringes 22 passend eingesetzt und seine Stirnfläche kann mit der Grundfläche des Stufenabschnittes verklebt sein. Der konische Teil 15b des Schlauchkörpers 15 ist mit einer zentralen kreisförmigen Öffnung 35 versehen, in die der Stützteil 33 des Käfigs 13 abgedichtet hineinragt. Der Schlauchkörper 15 wird zwischen dem Stützteil 33 und dem Stufenabschnitt 24 eingespannt, so daß er mit einer gewissen Vorspan nung radial gegen die Axialrippen 29 des Käfigs 13 angedrückt wird. Diese Vorspannung versteift den Schlauchkörper 15 und ermöglicht ein präzises Einstechen der Stahlkanüle einer Injektionsspritze in den Schlauchkörper 15 zur Einspritzung eines flüssigen Medikamentes o.dgl. in den von dem Schlauchkörper 15 umschlossenen Innenraum 40, der über den Durchlaßkanal 26 mit dem zum Patienten führenden Leitungsstrang IIb in offener Verbindung steht.

Der Schlauchkörper 15 ist zur Zuspritzung ringsum zugänglich, so daß die Leitung IIa,IIb nicht verdreht wird und ein kontinuierlicher Flüssigkeitsstrom gewährleistet ist.

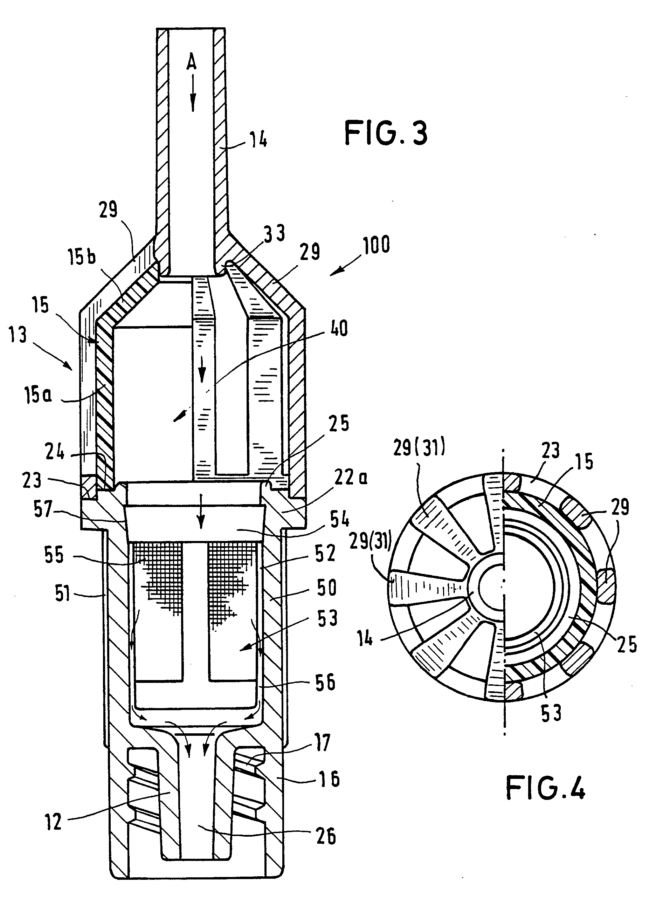

Das ebenfalls als endständiges Zwischenglied verwendbare Injektionsstück 100 gemäß Fign. 3 und 4 entspricht in seinen wesentlichen Teilen dem Injektionsstück 10 nach Fign. 1 und 2. Abweichend ist an die Gewindehülse 16 ein koaxiales zylindrisches Gehäuseteil 50 einstückig angeformt, das innen glattflächig ist und auf dessen Außenfläche parallele gerade Längsstege 51 angeordnet sind, die der Handhabungserleichterung dienen. Das Gehäuseteil 50 weist einen kreiszylindrischen Hohlraum 52 auf, der mit dem Innenraum 40 des Schlauchkörpers 15 und mit dem Durchlaßkanal 26 des Außenkonus 12 offen verbunden ist. In dem Hohlraum 52 hängt ein hohlzylindrischer Flächenfilter 53 beliebiger geeigneter Maschenweite. Der Flächenfilter 53 besteht aus einem Rahmen 54, der zwei halbzylindrische Maschenschalen 55 trägt und der so ausgebildet und angeordnet ist, daß die in Richtung der Pfeile A strömende Flüssigkeit axial in den Flächenfilter 53 eintritt und ihn radial verläßt, wobei sie durch einen schmalen Zwischenraum 56 zwischen der Innenwand des Gehäuseteils 50 und dem Flächenfilter 53 hindurch in den Durchlaßkanal 26 des Außenkonus 12 gelangt.

Zur hängenden Verbindung des Flächenfilters 53 mit dem Gehäuseteil 50 ist der obere offene Randteil des Rahmens 54 als in Durchflußrichtung kegelförmig verjüngter Ring ausgebildet, der in einer entsprechenden Umfangsausnehmung 57 des verbreiterten Profilringes 22a formschlüssig eingeklemmt ist. Durch Verklebung dieser Teile kann eine zusätzliche Sicherung des Flächenfilters 53 in zentrierter Position innerhalb des Hohlraumes 52 des Gehäuseteils 50 erreicht werden.

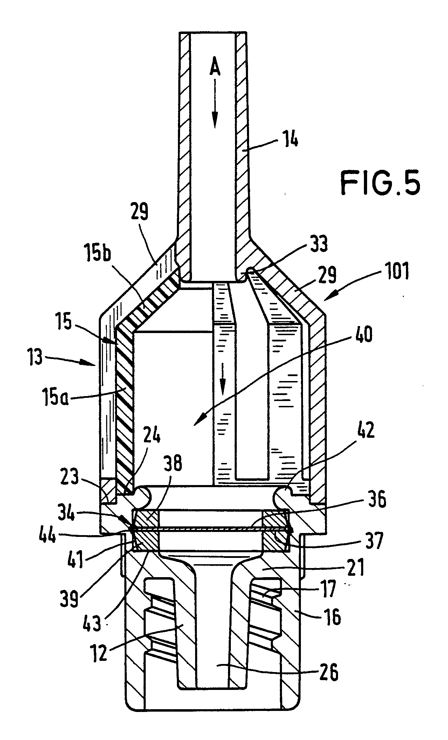

Fig. 5 zeigt ein Injektionsstück 101, dessen den Käfig 13 und den Schlauchkörper 15 betreffende Teile mit dem Injektionsstück 100 gemäß Figuren 3 und 4 übereinstimmen. Anders als bei diesem Beispiel ist ein Filter 34 ausgebildet und angeordnet. Der Filter 34 besteht vorteilhafterweise aus einem flachen, runden Gewebeteil 36, dessen kreisförmiger äußerer Rand 37 zwischen ebenen Flächen von zwei Ringen 38, 39 eingeklemmt ist. Jeder Ring 38, 39 hat kreiszylindrischen Innenumfang und konischen Außenumfang. Die beiden Ringe 38, 39 werden mit entgegengesetzt gerichteten Verjüngungen an beide Seiten des Gewebeteiles 36 angesetzt und in einem zylindrischen Gehäuseteil 41 zwischen dem Käfig 13 und dem Außenkonus 12 festgespannt. Zur Festspannung wird die aus den beiden Ringen 38, 39 und dem Gewebeteil 36 bestehende Einheit von einer Klemmwulst 42 an der Auslaßseite des Innenraumes 40 gegen eine Ringschulter 43 an der Basis 21 des Außenkonus 12 gepreßt. Dabei wird die zentrale spitze Rippe 44 an der Fuge zwischen beiden Ringen 38, 39 in das Material der Wand des Gehäuse teils 41 hineingedrückt, wodurch eine zusätzliche Zusammenpreßkraft auf den Gewebeteil 36 wirkt.

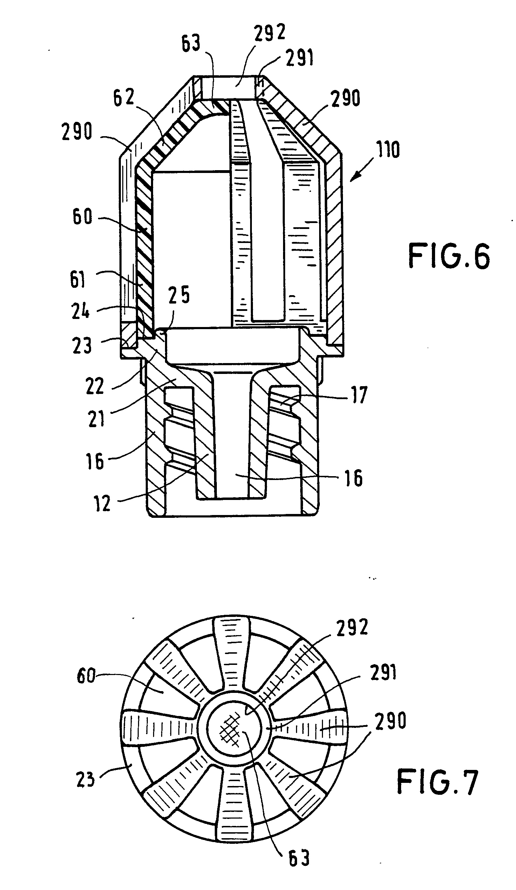

Das Beispiel der Fign. 6 und 7 veranschaulicht ein Injektionsstück 110, das als Verschlußstopfen für eine zu einem Patienten führende Leitung verwendbar ist. Auch in diesem Falle ist ein Außenkonus 12 mit Durchlaßkanal 26 vorgesehen, und es ist über eine Basis 21 eine Gewindehülse 16 mit dem Außenkonus 12 einstückig verbunden. Auch die Unterteilung des Profilringes 22 in zwei konzentrische Stufenabschnitte 23 und 24 wurde beibehalten. Der innere Stufenabschnitt 24 ist innen von der symmetrisch gewölbten Ringwulst 25 begrenzt.

Abweichend von den drei anderen Beispielen, die einen an beiden Enden offenen Schlauchkörper benutzen, ist bei dem Injektionsstück 110 der becherförmige Schlauchkörper 60 mit geschlossenem Boden 63 gestaltet. Der gummielastische Schlauchkörper 60 besteht aus einem kreiszylindrischen Teil 61, dessen unterer Rand passend in den Stufenabschnitt 24 eingreift und mit diesem fest verbunden ist, aus einem sich verjüngenden, kegelförmigen Teil 62 und aus einem kuppelförmigen, geschlossenen Boden 63. Die Teile 61 und 62 des Schlauchkörpers 60 liegen formschlüssig gegen die Innenfläche von Axialrippen 290 an, deren obere Enden an einen Ring 291 einstückig angreifen, dessen kreisförmige Öffnung 292 den Boden 63 des Schlauchkörpers 60 nach außen freilegt. Dieses Injektionsstück 110 ist insbesondere für Venenpunktionssysteme zur intermittierenden Injektion und wiederholten Blutentnahme geeignet, wobei es besonders günstig ist, daß das Injektionsstück 110 ein Durchstechen des Schlauchkörpers 60 mit Hilfe einer Stahlkanüle nicht nur an dem gesamten Umfang des Schlauchkörpers 60, sondern auch an seiner Scheitelzone ermöglicht. Die Größe der zum Durchstechen verfügbaren Schlauchkörperfläche ermöglicht bei häufigem Durchstechen des Schlauchkörpers 60 Einstiche an jeweils anderer Stelle, so daß ein perfekter Wiederverschluß jeder Perforationsöffnung gewährleistet ist und Undichtigkeiten ausgeschlossen sind. Da die zusätzliche Anbringung eines Filters, ähnlich den Beispielen gemäß Fign. 3 bis 5, auch bei dem als Verschlußstopfen benutzten Injektionsstück 110 möglich ist, gelingt auch bei dieser Anwendung eine Zurückhaltung von Partikeln aus der zum Patienten führenden Leitung.

**Ansprüche**

1. Injektionsstück für eine Leitung eines medizinischen Flüssigkeitsübertragungssystems mit einem Hohlkörper, der einen Wandteil aus durchstechbarem, gummielastischem Material aufweist und mit einem Anschlußkonus mit Durchlaßkanal zur Verbindung mit einem Gegenelement der Leitung versehen ist,
**dadurch gekennzeichnet,** daß der Anschlußkonus (12) mit einem korbartigen, durchbrochen ausgebildeten Käfig (13) fest verbunden ist, der gegen den Wandteil in Form eines gegen den Durchlaßkanal (26) des Anschlußkonus (12) offenen, gummielastischen, perforierbaren Schlauchkörpers (15) abstützend anliegt.

2. Injektionsstück nach Anspruch 1, dadurch gekennzeichnet, daß der Käfig (13) becherförmig mit mehreren umfangsmäßig verteilten Axialrippen (29) ausgebildet ist, und daß der Schlauchkörper formschlüssig in den Käfig (13) eingespannt ist.

3. Injektionsstück nach Anspruch 1 oder 2, dadurch gekennzeichnet, daß der Käfig (13) an seinem dem Anschlußkonus (12) gegenüberliegenden Ende einen Konnektorstutzen (14) aufweist und daß in dem dem Konnektorstutzen (14) zugewandten Ende des Schlauchkörpers (15) eine Öffnung (35) ausgebildet ist.

4. Injektionsstück nach Anspruch 1 oder 2, dadurch gekennzeichnet, daß der Schlauchkörper (60) an seinem dem Anschlußkonus (12) abgewandten Ende (63) geschlossen ausgebildet ist.

5. Injektionsstück nach einem der Ansprüche 1 bis 4, dadurch gekennzeichnet, daß zwischen dem Anschlußkonus (12) und dem Schlauchkörper (15) ein Filter (53) angeordnet ist.

6. Injektionsstück nach Anspruch 5, dadurch gekennzeichnet, daß der Filter (53) als hohlzylindrischer Flächenfilter ausgebildet und in einem mit dem Anschlußkonus (12) verbundenen Gehäuse (50) untergebracht ist.

FIG.1

FIG.2

FIG. 3

FIG. 4

FIG.5

FIG.6

FIG.7

## EINSCHLÄGIGE DOKUMENTE

EP 88103377.3

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | <u>EP - A1 - 0 111 723</u> (INTERMEDICAT)<br><br>* Fig.; Seite 1, 1. Absatz; Seite 5, Zeilen 3-15; Seite 5, Zeile 28 - Seite 6, Zeile 18 *<br><br>-- | 1 | A 61 M 5/31<br>A 61 M 5/14 |
| A | <u>DE - A1 - 2 634 368</u> (ERIKA INC.)<br><br>* Fig. 1-5; Seite 3, letzter Absatz - Seite 4, Zeile 6; Seite 5, 1. Absatz *<br><br>---- | 1 | |

|  |
|---|
| RECHERCHIERTE SACHGEBIETE (Int. Cl.4) |
| A 61 M 5/00 |
| A 61 M 25/00 |

Der vorliegende Recherchenbericht wurde für alle Patentansprüche erstellt.

| Recherchenort | Abschlußdatum der Recherche | Prüfer |
|---|---|---|
| WIEN | 08-09-1988 | LUDWIG |